# EUROPEAN PATENT APPLICATION

(11) **EP 2 749 321 A2**
(43) Date of publication of application: **02.07.2014**
(21) Application number: 13195291.3
(22) Date of filing: 02.12.2013
(51) Int. Cl.: A61Q 5/06, A61K 8/02, A61K 8/73

(54) **Dried hair styling compositions and products and methods for making dried personal care compositions**

(30) Priority: 28.12.2012 US 201213730678
(71) Applicant: The Dial Corporation, Scottsdale, AZ 85255 (US)
(72) Inventor: Harris, Jennifer, Arizona 85022, USA, Phoenix, (US); Demson, Robert, Creek, Arizona 85331, USA, Cave (US)
(74) Representative: Semrau, Markus

(57) **Abstract**

Hair styling compositions, hair styling products, and methods for making personal care compositions are provided herein. In one embodiment, a dried hair styling composition comprises a water-soluble solid film having a moisture content of less than about 10 wt%. The water-soluble solid film is adapted to be rehydrated to a flowable form when wetted in the hands of a user for application to the hair.

## Description

### TECHNICAL FIELD

The technical field relates generally to personal care compositions and methods of making personal care compositions, and more particularly relates to dried compositions and methods for making dried compositions.

### BACKGROUND

Today, an attractive hairstyle is generally regarded as an essential part of a well groomed appearance. Based on fashion trends, hairstyles that are considered chic can, for many types of hair, only be formed or sustained over a longer period of up to several days by using certain styling or fixing materials. Thus, hair treatments that provide permanent or temporary hair styling play an important role. Temporary styling products are intended to provide a good hold without compromising the healthy appearance of the hair, such as gloss.

Typically, one of the most important properties of a composition for the temporary styling of hair is providing the styled hair with the strongest possible hold for the style created. Styling hold is basically determined by the type and quantity of film-forming and/or setting agents used in the temporary styling product; however, other components of the styling product may also influence the hold. In addition to a high degree of hold, styling products must fulfill a series of additional requirements related to properties on the hair (e.g., moisture resistance, low stickiness, and a balanced conditioning effect), properties of the formulation in question (e.g., properties of the foam, gel, or sprayed aerosol), and properties concerning the handling of the styling product.

Conventional temporary hair styling compositions are in the form of, for example, hairsprays, hair waxes, hair gels, hair foams, setting lotions etc. One challenge with conventional temporary hair styling compositions is that each of these forms is a liquid or gel that must be housed by a relatively moisture-tight container. In addition, such liquid or gel can spill, causing mess on the user or in a suitcase during travel. Further, even in small "traveler" sizes, the containers generally hold enough temporary hair styling composition for many applications.

Accordingly, it is desirable to provide temporary hair styling compositions that provide sufficient styling hold in a dried form. It also is desirable to provide hair styling compositions in the form of single use dried strips that can be rehydrated to an aqueous flowable form when wetted by the user. Also, it is desirable to provide hair styling products that house single use dried strips in a non-moisture-tight container. In addition, it is desirable to provide methods for making such personal care compositions. Furthermore, other desirable features and characteristics will become apparent from the subsequent detailed description and the appended claims, taken in conjunction with the accompanying drawings and the foregoing technical field and background.

### BRIEF SUMMARY

Hair styling compositions, hair styling products, and methods for fabricating personal care compositions are provided herein. In an exemplary embodiment, a dried hair styling composition comprises a water-soluble solid film having a moisture content of less than about 10 weight percent (wt%). The water-soluble solid film is adapted to be rehydrated to a flowable form when wetted in the hands of a user for application to the hair.

In accordance with another exemplary embodiment, a hair styling product is provided. The hair styling product includes a container and a plurality of dried water-soluble solid strips of film housed in the container. Each strip of dried water-soluble solid film has a moisture content of less than about 10 wt%, is adapted to dissolve when wetted in the hands of a user for application to the hair, and includes a dried hair styling agent.

In accordance with another exemplary embodiment, a method for making a personal care composition in the form of a solid film is provided. The method includes mixing components including water into a flowable mixture having an initial moisture content. Further, the method includes casting the flowable mixture onto a substrate to form a cast mixture. Also, the method includes reducing the initial moisture content of the cast mixture by at least about 50% to form the solid film.

### BRIEF DESCRIPTION OF THE DRAWING

Embodiments of hair styling compositions, hair styling products, and methods for making personal care compositions will hereinafter be described in conjunction with the following drawing figure wherein:
FIG. 1 is a perspective view of a personal care composition in product form in accordance with an exemplary embodiment;
FIG. 2 is a cross sectional view of the personal care product of FIG. 1 in accordance with an exemplary embodiment; and
FIG. 3 is a perspective view of a personal care composition in product form in accordance with another exemplary embodiment.

### DETAILED DESCRIPTION

The following detailed description is merely exemplary in nature and is not intended to limit the hair styling compositions, hair styling products, and methods for making personal care compositions described herein. Furthermore, there is no intention to be bound by any expressed or implied theory presented in the preceding technical field, background or brief summary, or in the following detailed description.

The various embodiments contemplated herein relate to personal care compositions, and particularly to temporary hair styling compositions, in the form of dried water-soluble solid films. As used herein, "dried" refers to compositions, mixtures or products from which water, alcohols and/or other volatile solvents have been removed to form a substantially solid form. The dried water-soluble solid films may be packaged for single use by a user. Specifically, the user may remove a single dried film, rehydrate it by wetting it in his hands, and apply the rehydrated composition to his hair to provide hold for styling. An exemplary dried film has a moisture content of less than about 10 weight percent (wt%), for example less than about 5 wt%, for example less than about 2 wt%, such as less than about 1 wt%.

In an exemplary embodiment, the dried film is formed by first preparing a flowable mixture including hair styling agents and other components. After preparing the uniform mixture, it is cast onto a substrate to form a cast mixture. Moisture, in the form of water, alcohols or other solvents, is then removed from the cast mixture to form the dried film. The dried film may be cut into strips or pieces of a desirable dimension appropriate for a single use by a user.

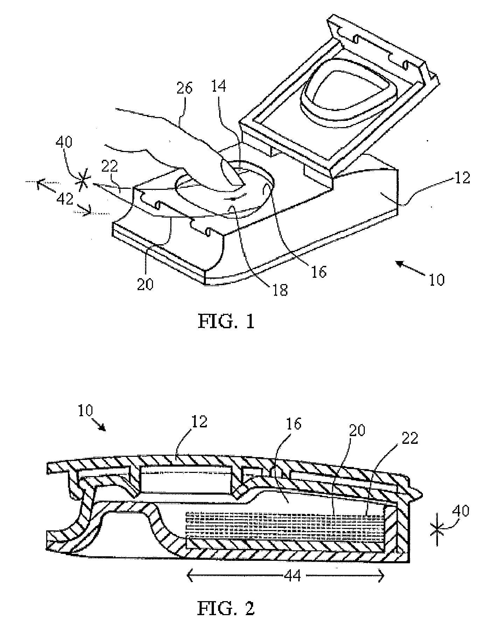

FIG. 1 illustrates an exemplary personal care product 10. As shown, the product 10 includes a container 12 with an opening 14 to a cavity 16. The cavity 16 holds a personal care composition 18 in the form of a plurality of strips 20 of dried water-soluble solid film 22. In an exemplary embodiment, the personal care composition 18 is a temporary hair styling composition. As shown, when the container 12 is in an opened configuration, a user 26 may remove a single strip 20 of dried film 22 through the opening 14 in the container 12 for a single use in styling the user's hair.

In FIG. 1, each strip 20 of dried film 22 has a thickness 40, a width 42, and a length 44 (not identified). The thickness 40, width 42, and length 44 are such that the strip 20 contains enough personal care composition 18 for a single styling application. In an exemplary embodiment, the thickness 40 of the film 22 is less than about 10 mm, for example less than about 3 mm, such as less than about 1.5 mm or less than about 1.0 mm. In another exemplary embodiment, the thickness 40 the film 22 is less than about 0.5 mm, for example less than about 0.25 mm, such as less than about 0.15 mm, less than about 0.1 mm, or less than about 0.05 mm. It is contemplated herein, that the thickness 40 of the film 22 can be engineered to provide a desirable amount of personal care composition 18 for a single use by the user 26. However, the film 22 must have a minimum thickness such as greater 0.001 mm to retain structural integrity. The width 42 of each strip 20 can be adjusted according to the desired use. While virtually any width can be used, it is envisioned that the product 10 can be carried unobtrusively in the user's pocket. Therefore, the width 42 may be similar to that of a stick of chewing gum, such as about 1.25 cm to about 2.5 cm.

FIG. 2 provides a cross sectional view of the container 12 in a closed configuration. As shown, a plurality of strips 20 of film 22 is held in the cavity 16 of the container 12. Each strip 20 has length 44 which, similar to the width 42, can be adjusted to virtually any desired measurement. However, in providing an unobtrusive and easily stored product, the length 44 may be about 2.5 cm to about 7.5 cm.

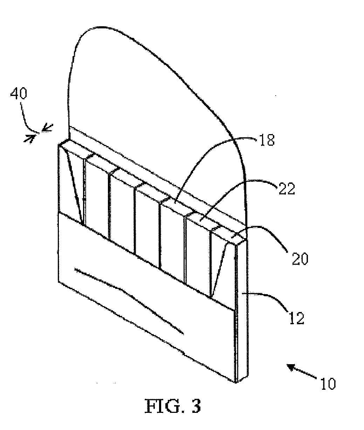

FIG. 3 illustrates another embodiment of the personal care product 10. In FIG. 3, the container 12 is constructed with paper. It is noted that the container 12 need not be water-tight, as the personal care composition 18 is dried and need not be completely contained and encased as aqueous, alcoholic or aqueous-alcoholic solutions in liquid or gel form must be.

The personal care composition 18 of the dried solid films 22 of FIGS. 1-3 is now described. In an exemplary embodiment, the flowable mixture used to form the dried solid film 22 includes at least one temporary hair styling agent in an aqueous, alcoholic or aqueous-alcoholic medium.

### Hair Styling Agent

In an exemplary embodiment, the temporary hair styling composition includes at least one temporary hair styling agent, such as a film-forming and/or setting agent. In certain embodiments, more than one hair styling agent is used.

Various synthetic polymers have been developed and are in use as styling agents. These polymers can be subdivided into cationic, anionic, non-ionic and amphoteric film-forming and/or setting polymers. Ideally these polymers form a polymer film when applied to hair, imparting a strong hold to the hairstyle while also being sufficiently flexible so as to not break under stress. If the polymer film is too brittle, film plaques develop (i.e., residues that are shed with movement of the hair and give the impression that the user of the respective styling agent has dandruff). In order to impart a strong hold, the setting polymer has to adhere well to the hair and form a sufficiently hard film. However, the resulting polymer film should not give the perception of being stiff to the collective hairs, but rather impart a degree of flexibility to the hair without losing the marked styling of the collective hairs (i.e., the hair style).

The flowable mixture may include synthetic, non-ionic hair fixing polymers such as homo- or copolymers of at least one monomer selected from vinyl pyrrolidone; vinyl caprolactam; vinyl ester (e.g., vinyl acetate), vinyl alcohol, acrylamide, methacrylamide, alkyl- and dialkyl acrylamide, alkyl- and dialkyl methacrylamide, dialkylaminoalkyl methacrylamide, dialkylaminoalkyl acrylamide, alkylacrylate, alkylmethacrylate, propylene glycol or ethylene glycol, wherein preferred alkyl groups of these monomers are C1- to C7-alkyl groups, more preferred C1- to C3-alkyl groups. Suitable are, e.g., homopolymers of vinyl caprolactam, homopolymers of vinyl pyrrolidone, homopolymers of N-vinyl formamide. Suitable hair fixing polymers are also copolymers of vinyl pyrrolidone and vinyl acetate; terpolymers of vinyl pyrrolidone, vinyl acetate and vinyl propionate; terpolymers of vinyl pyrrolidone, vinyl caprolactam and dialkylaminoalkyl(meth)acrylate; terpolymers of vinyl pyrrolidone, vinyl caprolactam and dialkylaminoalkyl(meth)acrylamide; polyacrylamide; polyvinyl alcohol; and hair fixing polyethylen glycol/polypropylen glycol copolymers; such as nonionic vinyl lactam homoor copolymers. Suitable vinyl lactams are, e.g., vinyl caprolactam and vinylpyrrolidone.

In an exemplary embodiment, the flowable mixture includes vinylpyrrolidone or vinyl acetate copolymers marketed as Luviskol® VA 73 W and polyvinylpyrrolidone (PVP) marketed as Luviskol® K90 Powder in 20% solution, both by BASF SE, of Ludwigshafen, Germany. For example, the flowable mixture may contain between from about 5 to about 15 wt%, such as about 10 wt%, vinylpyrrolidone or vinyl acetate copolymers, and about 15 to about 25 wt%, such as about 20 wt%, of a 20% solution of polyvinylpyrrolidone (PVP).

The flowable mixture may further include another film-forming and/or setting agent. For example, the composition may include a solution of vinylcaprolactam/ VP/dimethylaminoethyl methacrylate copolymer which is commercially available as Advantage® S from Ashland Inc. of Ashland, Kentucky. For example, the flowable mixture may include about 8 to about 15 wt%, such as about 12 wt%, vinylcaprolactam/VP/dimethylaminoethyl methacrylate copolymer in solution.

In an exemplary embodiment, the flowable mixture may further include as a film-forming and/or setting agent a copolymer of vinylpyrrolidone and dimethylaminopropyl methacrylamide commercially available as Styleze® CC-10 by Ashland Inc. For example, the flowable mixture may include about 5 to about 15 wt%, such as about 10 wt%, copolymer of vinylpyrrolidone and dimethylaminopropyl methacrylamide.

An exemplary flowable mixture further includes, as an additional or alternative film-forming and/or setting agent, a copolymer of vinylpyrrolidone and long-chain α-olefins, such as tricontanyl PVP. Tricontanyl PVP is commercially available as Antaron - Ganex® WP-660 from Ashland Inc. In an exemplary embodiment, the flowable mixture may include about 1 to about 2 wt%, such as about 1.5 wt%, tricontanyl PVP.

The flowable mixture may include an amphoteric polymer selected from the group of methacryloyl betaine/alkyl methacrylate copolymers, copolymers of monomers with carboxyl and/or sulfonic groups, in particular acrylic acid, methacrylic acid, itaconic acid and monomers with amino groups, in particular monoalkylaminoalkyl acrylates, dialkylaminoalkyl acrylates, monoalkylaminoalkyl methacrylates, dialkylaminoalkyl methacrylates, monoalkylaminoalkyl acrylique acid amides, dialkylaminoalkyl acrylique acid amides, monoalkylaminoalkyl methacrylique acid amides, dialkylaminoalkyl methacrylique acid amides and copolymers of N-octylacrylique acid amide, methyl methacrylate, hydroxypropyl methacrylate; N-tert-butylaminoethyl methacrylate and acrylic acid. For example, the amphoteric polymer may include an N-octylacrylamide/acrylic acid/tert-butylaminoethyl methacrylate copolymer.

An exemplary film-forming and/or setting amphoteric polymer may be contained in an amount of 0.01 to 20 wt%, for example 0.1 to 15 wt%, such as 1.0 to 10 wt%, based on the total flowable mixture. Multiple film-forming and/or setting amphoteric polymers may also be included, but in an exemplary embodiment, the total amount of film-forming and/or setting amphoteric polymers does not exceed 20 wt% of the flowable mixture.

Another film-forming and/or setting agent may include a copolymer such as polyquatemium-68 which is synthesized from vinylpyrrolidone, methacrylique acid amide, vinylimidazole and 3-methyl-1-vinylimidazolium methyl sulfate. Generally, such a copolymer includes a monomer selected from acrylique acid amide, methacrylique acid amide, N--C1-C10-alkylacrylique acid amide and N--C1-C10-alkylmethacrylique acid amide, a monomer from N-vinyllactams (such as N-vinylcaprolactam and N-vinylpyrrolidone), and a monomer selected from quaternized N-vinylimidazoles, and the monomer N-vinylimidazole (such as 3-methyl-1-vinylimidazolium methyl sulfate or salts of 3-alkyl-1-vinylimidazolium, in particular 3-(C1-C10-alkyl)-1-vinylimidazolium with physiologically acceptable anions including halides such as chloride, bromide and iodide, bicarbonate, bisulfate, monoalkyl sulfate, monomethyl sulfate and dihydrogen phosphate). An exemplary composition may contain one or a combination of such film-forming and/or setting agents in an amount of 0.01 to 20 wt% of the flowable mixture.

In addition to film-forming and/or setting agents discussed above, the composition may also contain other known film-forming and/or setting polymers. These film-forming and/or setting polymers may be either permanently or temporarily cationic, anionic or nonionic. Since polymers are often multifunctional, their functions cannot always be clearly and unambiguously demarcated from one other. This is true in particular of film-forming and setting polymers. However, it should be pointed out that both film-forming and setting polymers may be essential. However, since the two properties are not completely independent of one another, the term "setting polymers" is also always understood to include "film-forming polymers" and vice-versa.

In certain embodiments, beneficial properties of the film-forming polymers include the formation of a film. Film-forming polymers are understood to be polymers which, when dry, leave a continuous film on the skin, hair or nails. Such film-forming agents may be used in a wide variety of cosmetic products, e.g. face masks, makeup, hair-setting lotions, hair sprays, hair gels, hair waxes, hair treatments, shampoos or nail polish. The agent may include polymers having a sufficient solubility in water, alcohol or water/alcohol mixtures to be present in completely dissolved form in the flowable mixture. The film-forming polymers may be of synthetic or natural origin. Film-forming polymers are understood to include polymers which are capable of depositing a transparent polymer film on the hair when applied in 0.01 to 20 wt% aqueous, alcoholic or aqueous-alcoholic solutions.

Suitable additional film-forming, hair-setting polymers include, for example, homopolymers or copolymers synthesized from at least one of the following monomers: vinylpyrrolidone, vinyl caprolactam, vinyl esters, e.g. vinyl acetate, vinyl alcohol, acrylique acid amide, methacrylique acid amide, alkylacrylique acid amide and dialkylacrylique acid amide, alkylmethacrylique acid amide and dialkylmethacrylique acid amide, alkyl acrylate, alkyl methacrylate, propylene glycol or ethylene glycol, where the alkyl groups of these monomers are preferably C1 to C7 alkyl groups, particularly preferably C1 to C3 alkyl groups. Examples include the homopolymers of vinyl caprolactam, vinylpyrrolidone or N-vinylformamide. Other suitable synthetic film-forming, hair-setting polymers include, for example, copolymers of vinylpyrrolidone and vinyl acetate, terpolymers of vinylpyrrolidone, vinyl acetate and vinyl propionate, polyacrylique acid amides; polyvinyl alcohols; as well as polyethylene glycol/polypropylene glycol copolymers. Suitable natural film-forming polymers include, for example, cellulose derivatives, e.g. hydroxypropylcellulose with a molecular weight of 30,000 to 50,000 g/mol.

In an exemplary embodiment, the flowable mixture includes hydroxyethyl cellulose. Hydroxyethyl cellulose is commercially available as Cellosize™ Polymer PCG-10 from Amerchol Corporation of Edison, New Jersey, a subsidiary of Dow Chemical Company of Philadelphia, Pennsylvania. An exemplary flowable mixture includes about 0.15 to about 0.5 wt%, such as about 0.30 wt%, hydroxyethyl cellulose.

An exemplary flowable mixture may include another natural polymer, specifically a non-ionic, natural polymer based on modified corn starch. Such a natural polymer is commercially available as Amaze® from AkzoNobel of Amsterdam, Netherlands. In an exemplary embodiment, the flowable mixture includes about 0.25 to about 0.75 wt%, such as about 0.50 wt%, non-ionic, natural polymer based on modified corn starch.

Setting polymers contribute to the hold and/or to the creation of hair volume and to the body of the overall hair style. These so-called setting polymers are at the same time also film-forming polymers and therefore are typical substances in general for shaping hair treatment agents such as hair-setting agents, hair mousses, hair waxes, hair sprays. The formation of a film may join only a few hairs together, or may form over larger areas.

Substances that also impart hydrophobic properties to hair may be preferred because they reduce the tendency of hair to absorb moisture, i.e. water. This prevents strands of hair from hanging down limply and ensures a long-lasting set and hold. As polymers are multifunctional, they may also be successfully incorporated into leave-on and rinse-off hair treatments or shampoos.

An exemplary flowable mixture includes additional film-forming and/or setting polymers in an amount of 0.01 to 20 wt%, particularly 0.1 to 15 wt%, based on the total flowable mixture. Multiple film-forming and/or setting polymers may of course be included, but in an exemplary embodiment, the total amount of other film-forming and/or setting polymers does not exceed 20 wt%.

### Media

The flowable mixture may include water, such as demineralized water. In an exemplary embodiment, the flowable mixture includes about 25 to about 40 wt%, such as about 29 wt% water (not including water provided in polymer or other solutions).

The flowable mixture can include a polyol chosen from sorbitol, inositol, xylitol, mannitol, gluconolactone, glucuronic acid, 1,2,6-hexane triol, hydroxyethylsorbitol, phytantriol, hydroxypropylphytantriol, lactitol, maltitol, pentaerythritol, polyglycerin-3, glucose, lactose, maltose, polyglycerin-4, polyglycerin-6, polyglycerin-10, polyglycerin-20, polyglycerin-40, tripropylene glycol. The polyol may serve as a medium or excipient, and may also cooperate with a polymer to provide a strong styling hold and a flexible styling. For example, the flowable mixture may include a 70% solution of D-sorbitol in water. In an exemplary embodiment, the flowable mixture includes about 5 to about 15 wt%, such as about 10 wt%, of a 70% solution of D-sorbitol in water.

An exemplary flowable mixture may alternatively or additionally include petrolatum, or a homogeneous mixture of oily and waxy long-chain, nonpolar hydrocarbons, as an excipient and/or thickener. For example, a suitable petrolatum is commercially available as Perfecta® Petrolatum from Sonneborn Refined Products of Amsterdam, Netherlands. An exemplary flowable mixture may contain about 1 to about 5 wt%, such as about 2 wt%, petrolatum.

### Preservatives

The flowable mixture may further include preservatives. For example, the flowable mixture composition may include parabens, which are anti-microbial and antifungal. An exemplary paraben is methylparaben (methyl parahydroxybenzoate). In an exemplary embodiment, the flowable mixture includes about 0.08 to about 0.2 wt%, such as about 0.13 wt%, methylparaben. The flowable mixture may additionally or alternatively include propylparaben (4-Hydroxybenzoic acid propyl ester, propyl 4-hydroxybenzoate, or propyl paraben), which is active against a wide spectrum of fungi and bacteria at low concentrations. In an exemplary embodiment, the flowable mixture includes about 0.01 to about 0.05 wt%, such as about 0.03 wt%, propylparaben.

Further, the flowable mixture may include an antimicrobial formaldehyde releaser preservative. An exemplary preservative is DMDM hydantoin (1,3-Dimethylol-5,5-dimethylhydantoin), which is commercially available under the trade name Glydant® from Lonza of Basel, Switzerland. In an exemplary embodiment, the flowable mixture includes about 0.15 to about 0.5 wt%, such as 0.30 wt%, DMDM hydantoin.

An exemplary flowable mixture further includes a biocide preservative such as isothiazolinones. Isothiazolinones are commercially available as Kathon™ CG from Dow Chemical. Kathon™ CG contains 1.05 - 1.2% chloromethylisothiazolinone (CMIT); 0.3 - 0.45% methylisothiazolinone (MIT); 0.5 - 1.0% magnesium chloride, 21.0 - 23.5% magnesium nitrate, and 74.0 - 77.0% water. In an exemplary embodiment, the flowable mixture includes about 0.02 to about 0.1 wt%, such as about 0.05 wt%, Kathon™ CG.

### Emulsifiers

The flowable mixture may include one or more emulsifiers. For example, the flowable mixture may include cetearyl alcohol and dicetyl phosphate and ceteth-10 phosphate, which is commercially available as Crodafos™ CES from Croda of Snaith, UK. An exemplary flowable mixture includes about 1 to about 2 wt%, such as about 1.5 wt%, of Crodafos™ CES.

Further, the flowable mixture may include fatty alcohols. For example, the flowable mixture may include cetyl alcohol, such as that commercially available under ALFOL® 16NF from Sasol Ltd. of Johannesburg, South Africa. ALFOL® 16NF contains over 98% cetyl alcohol. An exemplary flowable mixture includes about 0.5 to about 1 wt%, such as about 0.75 wt%, cetyl alcohol.

Also, the flowable mixture may include the fatty alcohol stearyl alcohol, such as that commercially available under ALFOL® 18 from Sasol Ltd. ALFOL® 18 contains over 98% stearyl alcohol. An exemplary flowable mixture includes about 0.5 to about 1 wt%, such as about 0.75 wt%, stearyl alcohol.

Another exemplary fatty alcohol is polyethoxylated alcohol, which is a fatty alcohol derived from natural oils and fats. An exemplary polyethoxylated alcohol is Steareth-21 (Poly(oxy-1,2-ethanediyl)), which is a polyethylene glycol ether made from stearic acid. An exemplary flowable mixture includes about 0.5 to about 1 wt%, such as about 0.75 wt%, Steareth-21.

The flowable mixture may include an emulsifier such as beeswax. In exemplary embodiment, the composition includes PEG-8 beeswax, which is ethoxylated beeswax commercially available under the brand Apifil® from Gattefosse of Saint-Priest, France. In an exemplary embodiment, the flowable mixture includes about 0.15 to about 0.5 wt%, such as about 0.3 wt%, PEG-8 beeswax.

### Other Components

The flowable mixture contemplated herein also may comprise additives, such as those used in conventional personal care products. For example, in addition to styling efficacy, the mixture may include other additives that cause the applied product to exhibit long-lasting fragrance, odor protection, bacteria control, and/or another desired purpose and/or function. These additives include, but are not limited to, fragrances, including encapsulated fragrances, dyes, pigments, preservatives, antioxidants, moisturizers, and the like. These optional ingredients can be included in an amount of from about 0 to about 20 wt% of the flowable mixture. In exemplary embodiment, the flowable mixture includes about 0.2 to about 0.5 wt%, such as about 0.35 wt%, perfume.

The above list of materials is by way of example only and is not intended to be a comprehensive list of all potential components of the flowable mixture or dried styling composition contemplated herein. Other suitable components are readily identifiable to those skilled in the art and may be included as desired.

### Method of Making Personal Care Composition

An exemplary method for making the personal care composition in the form of a dried solid film includes mixing the selected components, including water, into a flowable mixture having an initial moisture content. In an exemplary embodiment, the initial moisture content is at least about 50%, such as at least about 60%. For example, the initial moisture content may be about 60% to about 80%.

The mixing process may involve the formation of more than one phase. For example, water, sorbitol, and methylparaben may be mixed to form a first phase. Separately, water and a film-forming and/or setting agent may be mixed to form a second phase. Further, water and another film-forming and/or setting agent or agents may be mixed to form a third phase. Other components may be mixed into one or more of the phases until uniform. Then, the separate phases are mixed together, either one-by-one or all three simultaneously. As a result of the mixing process, a flowable mixture is formed.

After formation of the flowable mixture, it is cast onto a substrate, such as a flat pan. In an exemplary embodiment, the cast mixture has a depth, or thickness, of about 1 mm to about 5 mm, for example from about 2 mm to about 4 mm, such as about 3 mm. Then, the cast mixture is dried. For example, the cast mixture can be heated to evaporate the water, alcohols, and any other moisture from the cast mixture.

It is preferred that the heating process be performed at moderate temperatures so that the components of the cast mixture are not damaged beyond being dried. For example, the heating process may be performed at about 100°C to about 180°C, such as at about 110°C to about 150°C, for example at about 120°C.

In an exemplary embodiment, the weight of the cast mixture is monitored during the heating process. As the moisture content of the cast mixture is reduced, the weight of the cast mixture necessarily decreases. When the weight of the cast mixture stabilizes for a duration of time, such as for 30 seconds, 60 seconds, or for another selected duration, then the cast mixture may be considered to be dried and established as the dried film. During an exemplary embodiment, the cast mixture is heated for about 15 to about 30 minutes, such as for about 20 minutes.

During the drying process, at least about 50% of the moisture is removed from the cast mixture. For example, at least about 66% of the moisture is removed, such as about 75% of the moisture, or about 90% of the moisture. In an exemplary embodiment, at least about 95%, at least about 99%, or substantially all of the moisture is removed from the cast mixture. As a result of the drying process, the dried film is formed with the desired thickness as discussed above in relation to FIG. 1.

### Method of Styling Hair with Strip of Dried Film

An exemplary method for styling hair with a strip of dried film includes obtaining a single strip of dried film by removing it from the product container. The user wets his hands either before or after obtaining the strip. The amount of water present on the user's hands may be selected by the user and adjusted through experience with prior hair styling operations. In an exemplary embodiment, the amount of water used may be about 0.5 mL to about 2 mL, such as about 1 mL.

Once the user holds the strip of dried film in his wetted hands, the user may rub or otherwise agitate the strip so that the water is absorbed by and hydrates the dried film. As the film becomes hydrated, it substantially returns to its fluid form and the user applies the fluid composition to his hair. After the user has styled his hair, the composition holds the hair in the desired styled form.

### Preferred embodiments

An especially preferred embodiment of the present invention is characterized by the following points:
1. A dried hair styling composition comprising a water-soluble solid film having a moisture content of less than about 10 wt%, wherein the film is adapted to be rehydrated to a flowable form when wetted in the hands of a user for application to the hair.
2. The dried hair styling composition according to point 1 wherein the water-soluble solid film comprises a dried hair styling agent and wherein the dried hair styling agent includes a dried synthetic, non-ionic hair fixing polymer.
3. The dried hair styling composition according to any of points 1 or 2 wherein the water-soluble solid film has a thickness of less than about 1.5 mm, preferably of less than about 1.0 mm, more preferably of less than about 0.5 mm, even more preferably of less than about 0.25 mm, most preferably of less than about 0.15 mm.
4. The dried hair styling composition according to any of points 1 to 3 wherein the water-soluble solid film has a moisture content of less than about 5 wt%.
5. The dried hair styling composition according to any of points 1 to 4 wherein the water-soluble solid film has a moisture content of less than about 1 wt%.
6. A hair styling product comprising:
   a container;
   a plurality of dried water-soluble solid strips of film according to any of points 1 to 5 housed in the container, wherein each strip of dried water-soluble solid film has a moisture content of less than about 10 wt%, is adapted to dissolve when wetted in the hands of a user for application to the hair, and includes a dried hair styling agent.
7. The hair styling product according to point 6 wherein the hair styling agent includes a dried synthetic, non-ionic hair fixing polymer.
8. The hair styling product according to any of points 6 or 7 wherein each strip of dried water-soluble solid film has a thickness of less than about 1.5 mm.
9. The hair styling product according to any of points 6 to 8 wherein each strip of dried water-soluble solid film has a moisture content of less than about 5 wt%.
10. A method for making a personal care composition in the form of a solid film according to any of points 1 to 5 comprising:
   mixing components including water into a flowable mixture having an initial moisture content;
   casting the flowable mixture onto a substrate to form a cast mixture; and
   reducing the initial moisture content of the cast mixture by at least about 50% to form the solid film.
11. The method according to point 10 wherein casting the flowable mixture onto a substrate forms the cast mixture with a thickness of about 3 mm.
12. The method according to any of points 10 or 11 wherein reducing the initial moisture content of the cast mixture forms the solid film with a thickness of no more than about 1.5 mm
13. The method according to any of points 10 to 12 wherein reducing the initial moisture content of the cast mixture comprises:
   monitoring the weight of the cast mixture; and
   heating the cast mixture until the weight stabilizes for a selected duration of time.
14. The method according to any of points 10 to 13 wherein reducing the initial moisture content of the cast mixture comprises heating the cast mixture to a temperature of about 100°C to about 180°C.
15. The method according to any of points 10 to 14 wherein reducing the initial moisture content comprises reducing the initial moisture content of the cast mixture by at least about 90% to form the solid film.

### Example

The following is an example of a flowable mixture before drying to form the dried film in accordance with an exemplary embodiment. The example is provided for illustration purposes only and is not meant to limit the various embodiments of the personal care product or composition in any way. All materials are set forth in weight percent.

Hair Styling Uniform Mixture - Pre-drying

| Ingredient | Wt% |
|---|---|
| Water | 28.79 |
| Sorbitol 70% solution | 10 |
| Methylparaben | 0.13 |
| Cellosize™ Polymer PCG-10 | 9.99 |
| Amaze® | 0.5 |
| Propylparaben | 0.03 |
| Perfecta® Petrolatum | 2 |
| Crodafos™ CES | 1.5 |
| ALFOL® 16NF | 0.75 |
| ALFOL® 18 | 0.75 |
| Antaron - Ganex® WP-660 | 1.5 |
| Steareth-21 | 0.75 |
| Apifil® | 0.3 |
| Luviskol® VA 73 W | 10 |
| Luviskol® K90 Powder in 20% solution | 20 |
| Advantage® S in solution | 12 |
| Styleze® CC-10 | 10 |
| Glydant® | 0.3 |
| Kathon™ CG | 0.05 |
| Perfume | 0.35 |
| Total | 100.0 |

During formation of the exemplary hair styling uniform mixture, 8.8 wt% of the water is mixed with the sorbitol and methylparaben to form a phase. Another 9.99 wt% of the water is mixed with the Cellosize™ Polymer PCG-10 to form a separate phase. The remaining 10 wt% of the water is mixed with the Amaze®, propylparaben and remaining components to form another phase. The first two phases are then mixed together to form an intermediate mixture before the third phase is added and mixed into the intermediate mixture to form the flowable mixture.

Accordingly, personal care compositions, such as hair styling compositions, in the form of dried films have been disclosed. Further, personal care products, such as hair styling products, are provided in the form of dried single use strips of film packaged in non-water-tight containers. Unlike the prior art, the exemplary embodiments taught herein disclose a dried film of a hair styling composition. During use, a strip of dried film may be hydrated by the user by rubbing the dried strip between the user's wetted hands. Further, the user may adjust the hold properties of the hair styling composition by adjusting the amount of water used during hydration.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the processes without departing from the scope defined by the claims, which includes known equivalents and foreseeable equivalents at the time of filing this patent application.

## Claims

1. A dried hair styling composition comprising a water-soluble solid film having a moisture content of less than about 10 wt%, wherein the film is adapted to be rehydrated to a flowable form when wetted in the hands of a user for application to the hair.

2. The dried hair styling composition of claim 1 wherein the water-soluble solid film comprises a dried hair styling agent and wherein the dried hair styling agent includes a dried synthetic, non-ionic hair fixing polymer.

3. The dried hair styling composition of claim 1 wherein the water-soluble solid film has a thickness of less than about 1.5 mm, preferably of less than about 1.0 mm, more preferably of less than about 0.5 mm, even more preferably of less than about 0.25 mm, most preferably of less than about 0.15 mm.

4. The dried hair styling composition of claim 1 wherein the water-soluble solid film has a moisture content of less than about 5 wt%.

5. The dried hair styling composition of claim 1 wherein the water-soluble solid film has a moisture content of less than about 1 wt%.

6. A hair styling product comprising:
a container;
a plurality of dried water-soluble solid strips of film housed in the container, wherein each strip of dried water-soluble solid film has a moisture content of less than about 10 wt%, is adapted to dissolve when wetted in the hands of a user for application to the hair, and includes a dried hair styling agent.

7. The hair styling product of claim 6 wherein the hair styling agent includes a dried synthetic, non-ionic hair fixing polymer.

8. The hair styling product of claim 6 wherein each strip of dried water-soluble solid film has a thickness of less than about 1.5 mm.

9. The hair styling product of claim 6 wherein each strip of dried water-soluble solid film has a moisture content of less than about 5 wt%.

10. A method for making a personal care composition in the form of a solid film comprising:
mixing components including water into a flowable mixture having an initial moisture content;
casting the flowable mixture onto a substrate to form a cast mixture; and
reducing the initial moisture content of the cast mixture by at least about 50% to form the solid film.

11. The method of claim 10 wherein casting the flowable mixture onto a substrate forms the cast mixture with a thickness of about 3 mm.

12. The method of claim 10 wherein reducing the initial moisture content of the cast mixture forms the solid film with a thickness of no more than about 1.5 mm

13. The method of claim 10 wherein reducing the initial moisture content of the cast mixture comprises:
monitoring the weight of the cast mixture; and
heating the cast mixture until the weight stabilizes for a selected duration of time.

14. The method of claim 10 wherein reducing the initial moisture content of the cast mixture comprises heating the cast mixture to a temperature of about 100°C to about 180°C.

15. The method of claim 10 wherein reducing the initial moisture content comprises reducing the initial moisture content of the cast mixture by at least about 90% to form the solid film.
